(19) **Europäisches Patentamt / European Patent Office / Office européen des brevets**

(11) **EP 3 128 323 B1**

(12) **EUROPEAN PATENT SPECIFICATION**

(45) Date of publication and mention
of the grant of the patent:
**06.06.2018 Bulletin 2018/23**

(51) Int Cl.:
*G01N 33/44* (2006.01)   *G01N 25/72* (2006.01)
*G06T 7/00* (2017.01)

(21) Application number: **15382421.4**

(22) Date of filing: **07.08.2015**

(54) **METHOD AND SYSTEM FOR DETECTING DEFECTS IN PLASTIC CONTAINERS**

VERFAHREN UND SYSTEM ZUR ERKENNUNG VON DEFEKTEN IN KUNSTSTOFFBEHÄLTERN

PROCÉDÉ ET SYSTÈME POUR DÉTECTER DES DÉFAUTS DANS DES RÉCIPIENTS EN PLASTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.02.2017 Bulletin 2017/06**

(73) Proprietor: **ainia**
**46980 Paterna (ES)**

(72) Inventors:
• **DÍAZ PUJOL, Ricardo**
  **46980 Paterna - Valencia (ES)**
• **BELENGUER BALLESTER, José**
  **46980 Paterna - Valencia (ES)**

• **GARCÍA REVERTER, José**
  **46980 Paterna - Valencia (ES)**
• **BLASCO PIQUER, Miguel**
  **46980 Paterna - Valencia (ES)**
• **SUBIRATS HUERTA, Sebastián**
  **46980 Paterna - Valencia (ES)**

(74) Representative: **ABG Patentes, S.L.**
**Avenida de Burgos, 16D**
**Edificio Euromor**
**28036 Madrid (ES)**

(56) References cited:
**WO-A1-2006/012194**

EP 3 128 323 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Object of the Invention

The present invention relates to a method and a system for detecting weld defects in plastic containers.

Background of the Invention

In plastic containers that are sealed by means of applying heat a weld is formed which sometimes may not fuse correctly, flaws therefore being formed in the weld which can cause the content of the container to deteriorate.

Inspection of the sealing zone can be performed in different ways, for example, by immersing the container in water or by means of palpation through operators, although this can be extremely costly and not completely reliable. Another method is based on shaking the container by means of vibrating machines, comparing the weight before and after shaking to check if there was product loss after shaking, but this method is only applicable in the cases in which the content of the container is powder or grain. Systems are also known to incorporate a tracer gas having the drawback of being slow, or to incorporate an ultrasound system with the drawback that only one weld, i.e., the weld that passes between the transducers, can be inspected, therefore being applicable only to the upper weld of flow-pack containers which are also known as "pillow" pack containers.

A more reliable method is infrared thermography, a technique which allows obtaining the temperature of a surface with precision without having any contact with said surface. A scope of application of analysis by means of thermography is analysis of heat sealing of plastic containers. An image of the material where the intensity of each point is proportional to the temperature of said point is obtained by means of sensors, such that temperature in the sealing zone can be monitored. However, this method does not allow determining if the weld has been suitably made and, therefore, if there will be defects in the sealing zone when said weld has cooled down.

Patent document JP2000088781 describes a technique using thermography for detecting defects in sealing, but the image analysis method is not very sensitive to flaws, as it corrects the image with respect to a standard and therefore does not contemplate changes in the boundary conditions such as sealing time, packaging speed, room temperature, etc.

Patent document WO2006012194 describes a method and apparatus for detecting defects in plastic containers by means of an infrared imaging system, although the method is not very reliable given that rejection of containers in the event of a defect is performed only if a series of extremely strict conditions is complied with, which can give rise to false positives in terms of valid containers.

Patent document JP2003307505 describes a correction unit for correcting the obtained images by means of room temperature. Room temperature is obtained through a temperature sensor. This has an advantage in inspecting the adhesion quality of the material, although the cooling of the surface of which the thermographic image is taken is not taken into account.

These prior art documents do not solve the technical problem of detecting in a sufficiently reliable and sensitive manner defects in the sealing zone because they do not take into account possible variables that may affect the temperature of the weld in the analysis after taking the thermographic image. Therefore, possible defects existing in the sealing zone are not detected, with the risk of considering welds with defects valid or of generating many false positives, increasing the production cost.

Description of the Invention

The technical problem described above is solved by means of the present invention, providing a method for detecting defects in a weld between two polymeric materials according to claim 1 and a system for detecting defects according to claim 9. Preferred embodiments of the invention are defined in the dependent claims.

The first inventive aspect of the invention relates to a method for detecting defects in a weld, obtained by heat-sealing, between two polymeric materials, comprising the following steps:

a) obtaining a thermographic image of the weld, in an instant $t_1$, wherein $t_0 < t_1 < t_{max}$, where $t_0$ is the time instant in which the weld has been completed, $t_{max}$ is the time instant in which the weld acquires room temperature ($T_{amb}$) after cooling down from a welding temperature ($T_{sold}$), wherein $T_{sold} > T_{amb}$, and $t_1$ is the instant in which the thermographic image is taken,

b) rescaling the thermographic image to increase the contrast between the zone corresponding to the weld and the rest of the container by correcting the value of each pixel based on the minimum and maximum temperature value of the image, obtaining as a result a reference image,

c) identifying in the reference image a weld area, binarizing the reference image and applying the binarized reference image by way of a mask on the thermographic image for identifying said weld area (S) in the thermographic image,
d) extracting at least one descriptor from the part of the thermographic image corresponding to the weld area, and
e) determining if the at least one extracted descriptor is within a validation interval defined by at least one threshold value,

wherein the method additionally comprises one of the following options:

i) correcting the thermographic image, before step d), at least the weld area, according to a value of the cooling of the polymeric materials taking into account the time elapsed between weld completion ($t_0$) and thermographic image capture ($t_1$), or
ii) correcting, between steps d) and e), the value of the at least one extracted descriptor, according to the value of the cooling of the polymeric materials taking into account the time elapsed between weld completion ($t_0$) and thermographic image capture ($t_1$), obtaining a corrected descriptor value, or
iii) correcting, before step e), the validation interval by correcting the at least one threshold value according to the cooling of the polymeric materials taking into account the time elapsed between weld completion ($t_0$) and thermographic image capture ($t_1$), The method for detecting defects according to the invention is envisaged for detecting defects in a container on which a heat-sealing operation has been performed, such that a weld or sealing zone closing the container is obtained.

The container is made up of two polymeric materials. Said materials can be simple materials (formed by a single compound) and complex materials (formed by the attachment of several materials). An example of complex materials is multilayer materials.

Step a: Obtaining a thermographic image

In step a) of the method for detecting defects, a thermographic image of the sealing zone is obtained, for example, with a thermographic camera. The thermographic image is obtained in time instant $t_1$. Instant $t_1$ is comprised in the interval [$t_0$, $t_{max}$], where $t_0$ is the instant in which sealing of the container is considered completed, and $t_{max}$ is the instant in which the temperature of the weld has become equal to the room temperature of the environment, and therefore the weld is said to have cooled down.
The thermographic image provides information relating to the temperature of each point of the object for subsequent analysis.

Step b: Rescaling the thermographic image to obtain a reference image for identifying a weld area

In step b) of the method for detecting defects, rescaling of the thermographic image obtained from the preceding step is performed to increase the contrast between the weld area and the rest of the container. To that end, the value of each pixel is corrected based on the minimum and maximum temperature value of the thermographic image, obtaining a reference image.
In particular embodiments, rescaling is performed according to the following expression:

$$T_{adjusted} = \frac{T - T_{min}}{T_{max} - T_{min}} \times (2^R - 1)$$

where R is the resolution defined as the number of bits used in digitizing the thermographic image, $T_{max}$ is the maximum temperature of the thermographic image, which is generally close to the welding temperature, T is the temperature of each point of the thermographic image, $T_{min}$ is the minimum temperature of the thermographic image, which is generally close to the room temperature, to the temperature of the materials not heated by welding or to the temperature of the product housed in the container and $T_{adjusted}$ is the temperature in the thermographic image rescaled to increase the contrast.
The rescaled image, herein referred to as "reference image", has a greater contrast between the weld area and the zones of the container further away from said weld area. Advantageously, increasing contrast in the rescaled image allows more directly and reliably identifying the weld area in successive steps of the method, which allows using the reference image by way of a mask to more easily identify the weld area in the thermographic image.

Step c: Identifying the weld area

The weld area has the same pattern associated with the contact zone between the jaw of the packaging machine with which the heat-sealing operation has been performed and the container. The perimeter of the weld area is identified in the reference image, for example, by means of a pattern identification algorithm. Once the weld area is identified, the reference image is binarized, comparing each pixel of the image with a binarization threshold value. This binarization involves reducing the information of the reference image to only two possible values; in case of a digital image, the values correspond to two colors: white for pixels having an intensity greater than the binarization threshold and black for pixels having an intensity less than the binarization threshold. One of the values corresponds to the identified weld area and the other value corresponds to the rest of the image.

By identifying said perimeter, the weld area corresponding to the sealing zone of the two polymeric materials is obtained in the reference image. The reference image comprises the points of interest for analysis and is applied like a mask on the original taken thermographic image to identify the weld area in the thermographic image. Said weld area of the thermographic image is formed by the set of points identified as the weld area in the reference image with the temperature information obtained in the thermographic image.

The identified weld area can be the complete sealing zone of the container or a part of the sealing zone, if analysis is to be performed only on said part of the sealing zone.

Step d: Extracting at least one descriptor

At least one descriptor is extracted from the part of the thermographic image corresponding to the weld area. A descriptor is a measurable physical parameter, representative of the quality of the weld, which allows comparing the magnitude thereof with reference data or with known data of an acceptable weld. The descriptors are established for boundary conditions $T_{amb}$, $T_{sold}$ and $t_1$ and can be, for example: the surface of the weld area, the width of the weld area, the maximum width of the weld area, the minimum width of the weld area, the mean temperature of the weld area, the minimum temperature in the weld area, the maximum temperature in the weld area, the perimeter of the weld area, the existence of at least one interruption in the weld area, as well as relations between these descriptors. An example of a descriptor relation would be obtaining percentages from the temperature of a part of the weld area with respect to the mean temperature of the complete weld area in order to compare the magnitude thereof with other reference percentages.

The descriptors may or may not be capable of being corrected according to the time elapsed between sealing and image capture. Descriptors that are not capable of being corrected according to the time elapsed between sealing and image capture are, for example, the surface of the weld area, the width of the weld area, the maximum width of the weld area, the minimum width of the weld area, the perimeter of the weld area and the interruption in the weld area. On the other hand, descriptors that are capable of being corrected according to the time elapsed between sealing and image capture are, for example, the mean temperature of the weld area, the minimum temperature of the weld area and the maximum temperature of the weld area.

Step e: Determining if the at least one extracted descriptor is within a validation interval defined by at least one threshold value

In this step, the value of the at least one descriptor obtained is compared with a validation interval defining the descriptor values for which the weld of the container is acceptable.

Under real conditions, the welding temperature $T_{sold}$, the room temperature $T_{amb}$ and/or the time elapsed between when the weld is made and the thermographic image is captured $t_1$ can change in successive heat-sealing operations. If the time in which the thermographic image is captured $t_1$ changes, the temperature in the weld zone of the thermographic image will also change due to cooling. To take this effect into account, the method of the invention takes into account the cooling of the polymeric materials of the weld in the time elapsed between weld completion and thermographic image capture, according to one of the following options:

    i. Correcting, before step d), the thermographic image, at least in the weld area, according to the value of the cooling of the polymeric materials in the time interval $t_1-t_0$, i.e., the time elapsed between weld completion and thermographic image capture.

    ii. Correcting, between steps d) and e), the value of at least one extracted descriptor, according to the value of the cooling of the polymeric materials in the time interval $t_1-t_0$, obtaining a corrected descriptor value.

    iii. Correcting, before step e), the validation interval by correcting the at least one threshold value according to the cooling of the polymeric materials in the time interval $t_1-t_0$.

Option i): Correcting the thermographic image according to boundary conditions

As mentioned above, variables such as temperature $T_{amb}$ of the environment of the container, or the time $t_1$ elapsed from the end of the sealing operation, affect evolution of the temperature of the weld. In this embodiment, the method of the present invention evaluates said variables and adjusts the thermographic image taken to obtain a reliable result of the existing defects. Said adjustment results in a corrected thermographic image, in which at least the zone of the image corresponding to the weld area has been corrected to take into account the cooling of the materials in the time elapsed between heat-sealing operation completion and thermographic image capture. Although correcting the part of the thermographic image corresponding to the weld area is sufficient, the correction can be made in the complete thermographic image, if desired.

When the method is performed according to this option, after correcting the thermographic image, the method continues with step d) for obtaining at least one descriptor from the weld area, and with step e) for determining if the descriptor or descriptors extracted from the thermographic image are within a defined validation interval.

Option ii): Correcting the descriptors according to boundary conditions

As mentioned above, the descriptors may or may not be capable of being corrected according to the time elapsed between sealing and image capture. When the descriptors used in the method are capable of being corrected according to the time elapsed between sealing and image capture, instead of correcting the thermographic image like in option i), the extracted descriptors can be corrected.

When the method is performed according to this option, the method proceeds with identifying the weld area in the original thermographic image and with obtaining at least one descriptor, as defined in steps c) and d). However, before comparing the at least one descriptor and the validation interval, the method proceeds with correcting the extracted descriptors to take into account the time elapsed between sealing completion and thermographic image capture. The corrected descriptors therefore correspond to the same capture instant considered for establishing the validation interval. The method then continues by comparing the corrected descriptors with the defined validation interval.

Option iii): Correcting the thresholds of the validation interval

As a variant of option ii), when at least one extracted descriptor can be corrected according to time, the validation interval can be corrected instead of correcting the descriptor.

When the method is performed according to this option, the at least one descriptor of the weld area of the thermographic image is obtained and before performing step e) of comparing the descriptor with the validation interval, correction of the at least one threshold defining the validation interval is carried out.

If the thresholds of the validation interval are capable of being corrected according to the time elapsed between sealing and image capture, when said correction is not made and the time $t_1$ elapsed from the end of the sealing operation is not that envisaged for said thresholds, the comparison between the descriptor and the validation interval may not be correct. The method of the invention according to option iii) adjusts the value of the thresholds of the validation interval, resulting in corrected thresholds for the time elapsed between sealing and image capture. According to this option, the method continues in step e), comparing the at least one descriptor with the validation interval corrected according to the time elapsed between sealing and image capture.

As mentioned, adjusting temperature according to the time $t_1$ elapsed from the end of the sealing operation can be carried out by correcting the thermographic image (option i)), by correcting the descriptors (option ii)), or by correcting the acceptable thresholds of the validation interval (option iii)). In a particular embodiment, the value of the cooling of the polymeric materials used in any of options i) to iii) is taken from a characteristic function (f) representing the cooling of the weld formed by the polymeric materials with respect to time, for an initial temperature $T_{sold}$ and room temperature $T_{amb}$.

Advantageously, the characteristic function (f) allows knowing the temperature of the sealing zone according to the time elapsed from sealing completion. This evolution of temperature is the cooling of the attachment of the polymeric materials, which can depend both on parameters characteristic of the materials and on parameters characteristic of the heat-sealing operation or of the room temperature.

In a particular embodiment, in the method for detecting defects according to the first inventive aspect the characteristic function (f) is obtained from an experimental characteristic curve formed for the weld of two polymeric materials, by means of recording the temperature values of the weld during the time interval $[t_0, t_{max}]$ for a given $T_{sold}$ and $T_{amb}$.

This allows obtaining the cooling for each particular example of combination of polymeric materials and heat-sealing operations. Advantageously, using the characteristic function (f) is reliable given that the behavior of the weld area can be extrapolated according to the evolution of the recorded temperatures with which each of the characteristic functions (f) has been formed. The characteristic function can be obtained as modeling based on the data of the experimental curve, or another numerical adjustment to a mathematical expression.

Furthermore, there are theoretical alternatives for obtaining the characteristic function (f) that allow reducing the time and cost of obtaining said function, avoiding the need to perform experimental testing with a large number of samples which allow forming said characteristic function (f).

The validation interval defined by at least one threshold value can be established before starting the implementation of the method. One way for establishing same can be by means of the comparative analysis of weld sample images with and without defects. In a particular embodiment, to obtain sample images with and without defect, samples are prepared in which the temperature of a jaw used for sealing the container and the welding time are changed, and flaws are purposely made by means of generating creases and wrinkles and by means of placing packaged product residues in the weld zone. The corresponding thermographic images are obtained and it is analyzed whether or not the container has caused leaks, for which purpose it is immersed in a liquid to check if there are leaks from inside the container. Based on samples that have caused a leak, the validation intervals of the descriptors for the established $T_{amb}$ and $T_{sold}$ for which the weld of the container is acceptable can be established.

Another particular embodiment for establishing the validation interval is by means of an experimental test with a representative sample set, for which purpose thermographic images are acquired for a period and the descriptors are extracted, the descriptors being recorded with and without correction according to the method of the invention. Based on statistical analysis and assuming that it follows a normal distribution, a 3σ criterion (or the like) is established, such that the acceptable limits for each descriptor are established in the interval $[-3\sigma, 3\sigma]$. Subsequently, when a container is rejected because a descriptor is outside that interval, the container is analyzed to see if the defect causes a leak for the purpose of adjusting the thresholds of the interval, lowering or raising them for that descriptor. Once a validation interval for each descriptor has been fixed, they can be used in the method of the present invention for analyzing the sealing zone of the container and detecting possible defects obtained during the heat-sealing process.

If the descriptor extracted from the thermographic image is not within the previously defined validation interval, a defect in the sealing zone is detected, so the container is not considered valid.

Advantageously, the method of the invention allows a more reliable detection of defects in the sealing zone. Furthermore, the method allows detecting possible flaws in the sealing system if said defects are repeated in successive heat-sealing operations.

In a particular embodiment, in the method for detecting defects according to the first inventive aspect, before step d) the weld area of the thermographic image is subdivided into a plurality of segments, and steps d) and e) are applied on each of the segments and the weld is classified as defective if a defect is detected in at least one of the segments.

Advantageously, subdividing the weld area identified in the thermographic image into segments allows a more thorough analysis of the sealing zone of the container, given that the acceptance criteria of the container are stricter since they must be met by a plurality of segments and not only by the unitary weld area. Furthermore, the jaws used for making the welds are generally not homogeneous, there being zones with different welding temperatures and widths, so working by segments allows increasing the flaw detection sensitivity.

In the case of dividing the weld area into a plurality of segments or subareas, an exclusion area can be established in those areas of the image in which there is no weld to reduce the computing cost and to process only the segments or subareas of interest, for performing the adjustment thereof, for extracting the descriptors and for comparing them to the limits of the corresponding validation intervals.

The present invention provides a decision method for determining whether or not a container is well sealed based on a multiparameter system for detecting containers the weld of which does not comply with quality standards.

The second inventive aspect of the invention relates to a system for detecting defects in a weld between two polymeric materials, comprising a thermographic camera, processing means and at least one time controlling means, in which the processing means are adapted for implementing a method for detecting defects according to the first inventive aspect.

The system comprises a thermographic camera adapted for taking thermographic images of the containers to be analyzed. The thermographic images obtained as a result provide the temperature of each point of the container present in the image based on measuring the infrared radiation emitted by the materials and on converting said radiation into information relating to temperature.

The system also comprises processing means which are adapted for managing data inputs obtained from the thermographic camera and for managing the control algorithms implemented for performing thermographic image analysis according to the method of the first inventive aspect.

The system also comprises time controlling means which allow recording both the instant $t_0$, or the instant in which the sealing of the container is considered completed, and the instant $t_1$, the instant in which the thermographic image of the container is taken by means of the thermographic camera, which allows measuring the time elapsed since heat is no longer applied to the container until the thermographic image is taken. The cooling experienced by the weld throughout this time interval is adjusted by the processing means.

In a particular embodiment, the system for detecting defects according to the second inventive aspect further comprises:

- a welding element adapted for making the weld,
- a conveyance system adapted for conveying the container from welding position P1 to position P2 in which the thermographic image is taken, and
- the time controlling means are two position sensors adapted for determining the time elapsed between weld completion when the container is in position P1 and the moment in which the container has reached position P2 in which the thermographic image is taken.

Advantageously, the two position sensors allow recording the passage of the container in two time instants of the process for subsequently making the correction on the thermographic image taken of the container according to the time $t_1$ elapsed from the end of the sealing operation to thermographic image capture.

Advantageously, an inspection system for inspecting the closure of plastic containers heat-sealed according to the invention allows evaluating weld quality by means of thermography and automatically correcting variations of the environment such as changes in room temperature and variations in the time elapsed from sealing to image capture.

In a particular case, the system comprises means for automatically ejecting containers having some sort of closure defect.

All the features and/or steps of the methods described in this specification (including the claims, description and drawings) can be combined in any combination, with the exception of the combinations of such mutually exclusive features.

Description of the Drawings

The foregoing and other features and advantages of the invention will be more clearly understood based on the following detailed description of a preferred embodiment provided only by way of illustrative and non-limiting example in reference to the attached drawings.

Figure 1 shows an example of a system for detecting sealing defects according to the invention.

Figure 2A shows an example of a thermographic image taken by the thermographic camera.

Figure 2B shows an example of an image rescaled based on a thermographic image.

Figure 2C shows an example of a mask after identifying the pattern of the weld.

Figure 2D shows an example of the resulting image after applying the mask on the thermographic image.

Figure 2E shows an example of the modified image with the points of the weld area corrected according to the time elapsed between sealing operation completion and thermographic image capture.

Figure 3 shows an example of a characteristic function (f) for a specific weld.

Figure 4A shows an example of a thermographic image of a container in which the weld area has been subdivided for analysis.

Figure 4B shows an example of a thermographic image of a container in which the weld area has been subdivided for analysis, the exclusion zone having been removed from the thermographic image.

Figure 5 shows an example of the curve depicting a drop in temperature of a weld for 120 seconds with $K = 0.042s^{-1}$, $T_{amb} = 34°C$ and $T_{sold} = 93.1°C$.

Figure 6 shows a collection of curves depicting a drop in temperature for a weld between a pair of polymeric materials with different $T_{sold}$ and $T_{amb}$.

Detailed Description of the Invention

The present invention relates to a method and a system for detecting defects in a weld obtained by heat-sealing between two polymeric materials. Said method analyzes a thermographic image taken of the sealing zone of the container based on different variables.

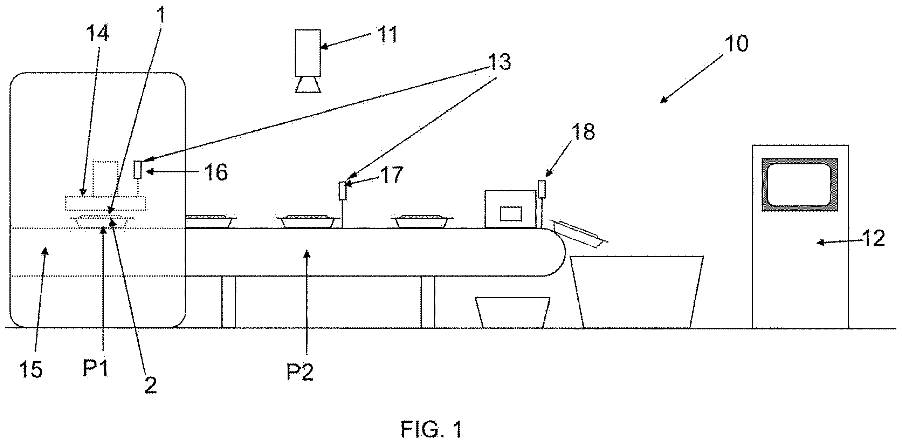

Figure 1 shows a system according to one embodiment of the invention, comprising a container sealing unit for sealing containers by means of applying heat and a thermographic image capturing system for capturing thermographic images for subsequent analysis. Said analysis allows evaluating weld quality and detecting possible defects by applying a method according to the first inventive aspect. Therefore, defective units can be rejected and the release of containers with leaks or defects in the sealing zone of the container is prevented.

In the particular example of Figure 1, the method is implemented in a system (10) comprising a welding element (14) or jaw and two time detection units (13) respectively recording the instant in which sealing is completed, i.e., the instant in which the jaw of the welding element (14) releases the container ($t_0$), and the instant in which the image of the

thermographic image ($t_1$) is captured.

In the example of Figure 1, the time controlling means (13) are two position sensors (16, 17) adapted for determining the time elapsed between the weld being completed and the container moving from position P1 to position P2 in which the thermographic image is taken.

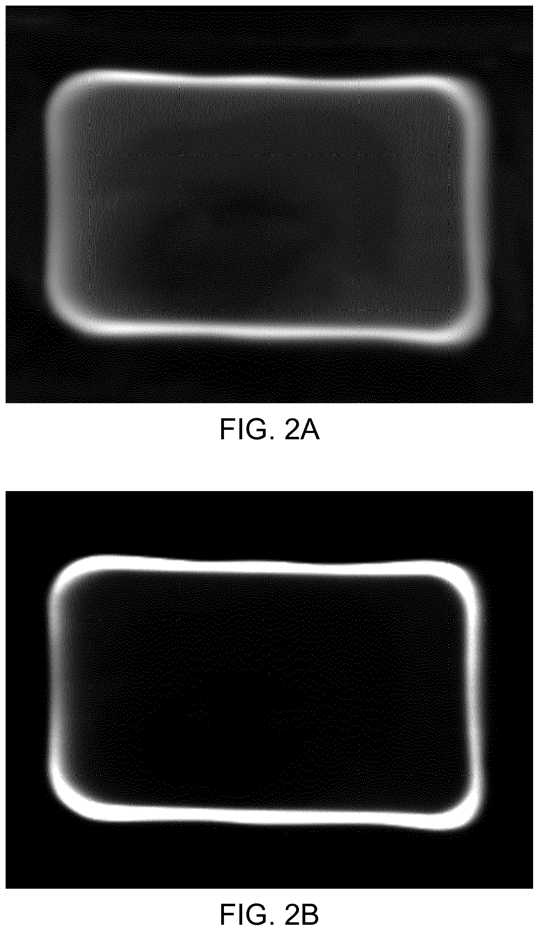

A thermographic camera (11) takes a thermographic image of the container and processing means (12) analyze the thermographic image. Figure 2a depicts the thermographic image (I) of the container. The conveyance system (15) in this case is a conveyor belt which allows automatically transferring containers from the sealing position to the position of the thermographic camera (11). In another embodiment, an alternative conveyance system could be used. In the particular example of Figure 1, the system (10) additionally comprises ejection means (18) for ejecting containers in which at least one defect has been detected, upon having identified that at least one descriptor is outside the thresholds of the validation interval which determines when a container is acceptable. If the container is rejected, the ejection means (18) detect the container to be rejected and eject it from the conveyor belt (15) .

In this embodiment, the room temperature of the environment where the thermographic image is taken is obtained by means of the thermographic camera (11) itself. In other embodiments, another temperature sensor could be used.

Figure 2A depicts the thermographic image (I) obtained of the container. Figure 2B shows the reference image (R) obtained by rescaling the contrast in the thermographic image by means of the following expression:

$$T_{adjusted} = \frac{T - T_{min}}{T_{max} - T_{min}} \times (2^R - 1)$$

where:

- R is the resolution defined as the number of bits used in digitizing the thermographic image in the thermographic camera,
- $T_{max}$ is the maximum temperature in the thermographic image,
- T is the temperature measured at a point of the weld area,
- $T_{min}$ is the minimum temperature in the thermographic image.

As seen when comparing Figures 2A and 2B, the rescaled image (also referred to as reference image) has greater contrast between the weld zone and the zones of the container further away from said weld zone than the original thermographic image. Said contrast increases when distributing the entire defined temperature value interval for rescaling according to the resolution of the thermographic camera (11), which can be 8 bits, 12 bits, 16 bits or more, between the minimum and maximum values of the thermographic image.

In a particular example, the images are rescaled for 16 bits and the minimum and maximum pixel intensity values corresponding to temperature are 27898 and 31554. After rescaling, the minimum and maximum values become 0 and 65535. The rescaled image is used for identifying the weld area and dividing it into subareas.

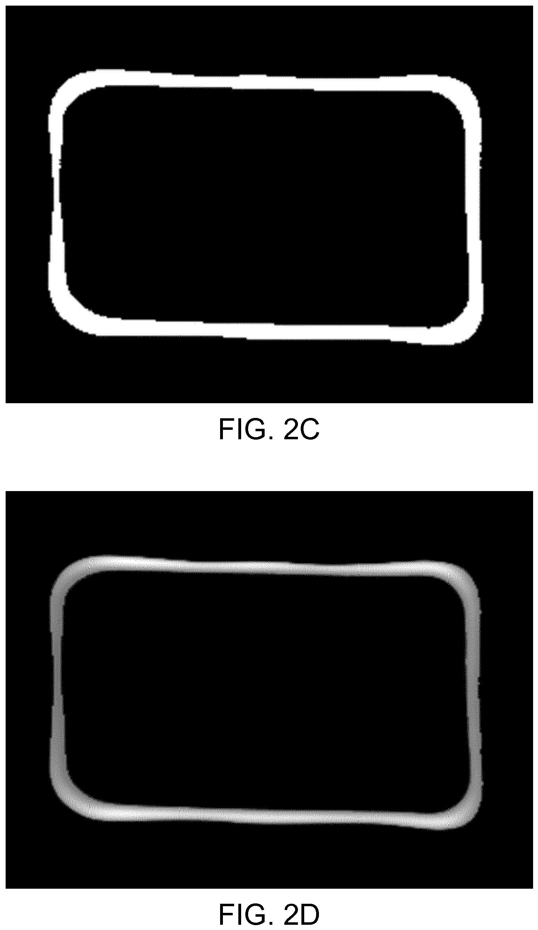

The weld area (S) is identified on the rescaled image (R) shown in Figure 2B. The rescaled image is binarized into two zones: one (white) zone corresponding to the weld area and another (black) zone corresponding to the rest of the container. Figure 2C shows said binarized image. The binarized image is used as a mask to be applied on the original thermographic image, such that in the resulting image, depicted in Figure 2D, the weld area with the temperature values of the original thermographic image taken in instant $t_1$ is obtained, whereas the rest of the image has a value of 0 and is depicted in black.

The temperature of each point of the weld area is obtained from the pixel value in image 2D and is calculated according to the expression:

```
Temperature(°C) =(Pixel intensity/100)-273.
```

This calculation is a particular example in which the temperature is considered proportional to the intensity of the sensor of the thermographic camera. This relation depends on the manufacturer of the thermographic camera and is internally corrected by means of

- $T_{amb}$,
- distance between object and sensor,
- relative humidity, and
- emissivity of the analyzed polymers.

In the particular example that has been described, the thermographic image is corrected according to the method of the invention to compensate for the cooling of the weld from its completion until thermographic image capture to take into consideration the actual time that elapsed until image capture.

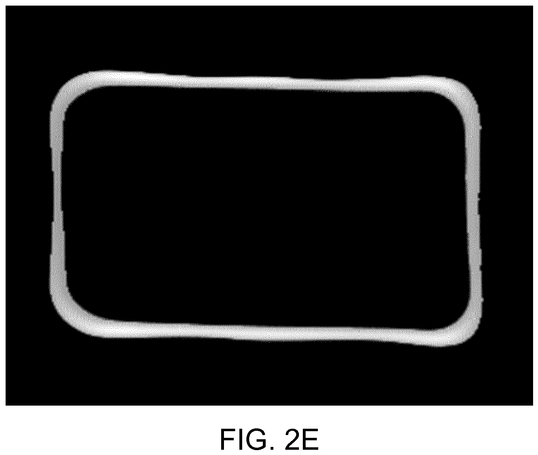

In the particular example that has been described, an adjustment is performed according to time $t_1$ taking into account the cooling of the heat-sealed polymeric materials (1, 2). The cooling of the weld formed by these polymeric materials (1, 2) is taken from a characteristic function (f) representing the cooling of the attachment formed after welding with respect to time. The result of the adjustment is the corrected image of Figure 2E, in which the points included in the weld area have been corrected to take into account the cooling experienced during the actual time that elapsed between sealing completion and thermographic image capture.

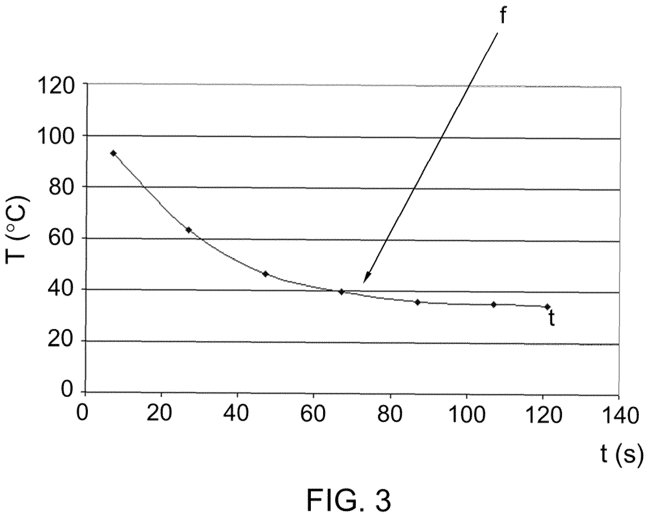

Figure 3 shows a possible characteristic function (f) or curve depicting the drop in temperature over time of an attachment of polymeric materials (1, 2).

In the particular example of Figure 3, the characteristic function (f) is obtained by means of an equation modeling the experimental characteristic curve of cooling of the materials (Newton's law of cooling). In other embodiments, other approaches or models can be used. The function can correspond to the following equation:

$$f(t) = T_{amb} + (T_{sold} - T_{amb})e^{-Kt}$$

wherein $T_{sold}$ is the initial temperature of the weld upon removing the jaw, $T_{amb}$ is the room temperature, and K is a constant characteristic of each complex of polymers (1, 2).

The temperature $T_{amb}$ can be measured with a temperature sensor and can be considered constant from the sealing of the container until it is inspected because the time elapsed is small enough. The temperature $T_{sold}$ can be obtained directly from the sealing machine. The constant K can be determined from experimental results of the drop in temperature over time, adjusting said data to the theoretical expression.

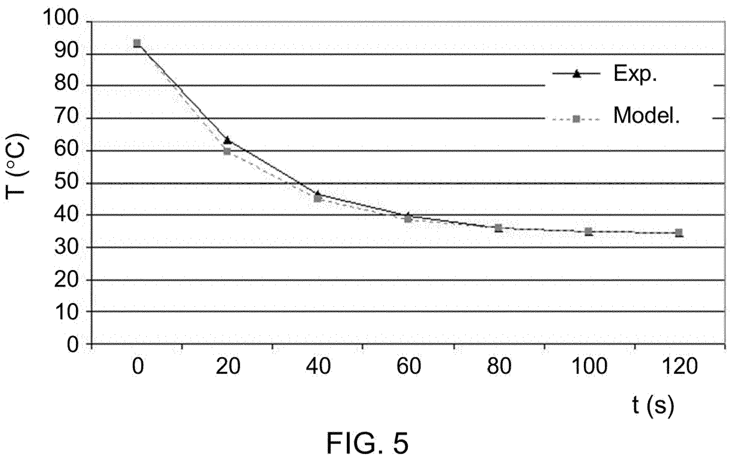

Figure 5 shows an example of a characteristic function of a weld for 120 seconds with K = 0.042 s$^{-1}$, $T_{amb}$ = 34°C and $T_{sold}$ = 93.1°C, a good adjustment being observed between the experimental curve and the theoretical model following Newton's law of cooling.

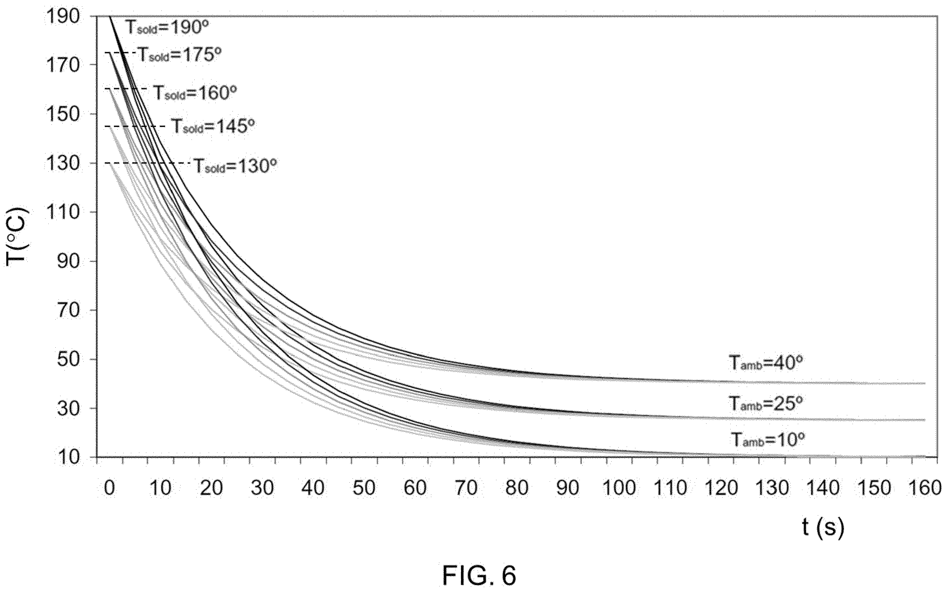

Figure 6 shows a collection of characteristic functions depicting the drop in temperature over time for a weld between a pair of polymeric materials. The depicted curves correspond to different combinations of 5 different welding temperatures $T_{sold}$ ($T_{sold}$ = 130°C, 145°C, 160°C, 175°C and 190°C) and 3 room temperature values $T_{amb}$ ($T_{amb}$ = 10°C, 25°C and 40°C) .

Experimental method for obtaining cooling curves

An experimental method for obtaining curves depicting the drop in temperature of the polymeric materials after applying heat comprises sealing the polymeric materials for a specific $T_{amb}$ and a specific $T_{sold}$, with a fixed sealing time, and recording the evolution of temperature over time in the sealing zone of the container once it is released from the sealing jaw for obtaining the curve depicting the drop. By repeating the method for different welding temperatures and different room temperatures, a set of experimental curves with different boundary conditions is obtained.

Experimental method for obtaining the constant K

With only one point i ($t_i$, $T_i$) of the temperature of a welding point $T_i$ for a time instant $t_i$ of the curve and based on Newton's law of cooling, the constant K for the drop in temperature of the polymeric materials (1, 2) can be obtained:

$$K = -\frac{1}{t_i} \cdot \ln \frac{(T_i - T_{amb})}{(T_{sold} - T_{amb})}$$

and the model of the drop in a system formed by a container and heat-sealing machine can be characterized to enable analyzing containers sealed by means of said system based on the defined constant K and for any $T_{sold}$ and $T_{amb}$ temperature.

As described, in processes in which several containers are sealed consecutively, for example, in a chain packaging system, the time $t_1$ since the weld is made until the image for each container is captured can be variable. According to the method of the invention, this effect can be taken into account in several ways:

- Adjusting the image: each point or pixel of the weld area in the thermographic image is corrected based on the characteristic cooling curve f(t) and on the new corrected image, the descriptor/descriptors to be analyzed is/are always extracted for one and the same time instant $t_1 < t_{max}$.

- Adjusting the descriptors capable of being corrected according to the time elapsed between sealing and image capture: for a temperature value of a descriptor or set of descriptors in an instant $t_1$, the values for that descriptor or set of descriptors are recalculated according to the characteristic cooling curve f(t) for one and the same time instant $t_1 < t_{max}$.
- Adjusting the temperature limits: for a mean temperature value of a point or of a set of points in an instant $t_1$, the limits for that point or set of points are recalculated according to the characteristic cooling curve f(t) for one and the same time instant $t_1 < t_{max}$.

In other words, when the time $t_1$ from which the weld is made until the image is captured is variable between several containers, a method according to the invention in which time $t_1 < t_{max}$ is taken as a reference is then applied.

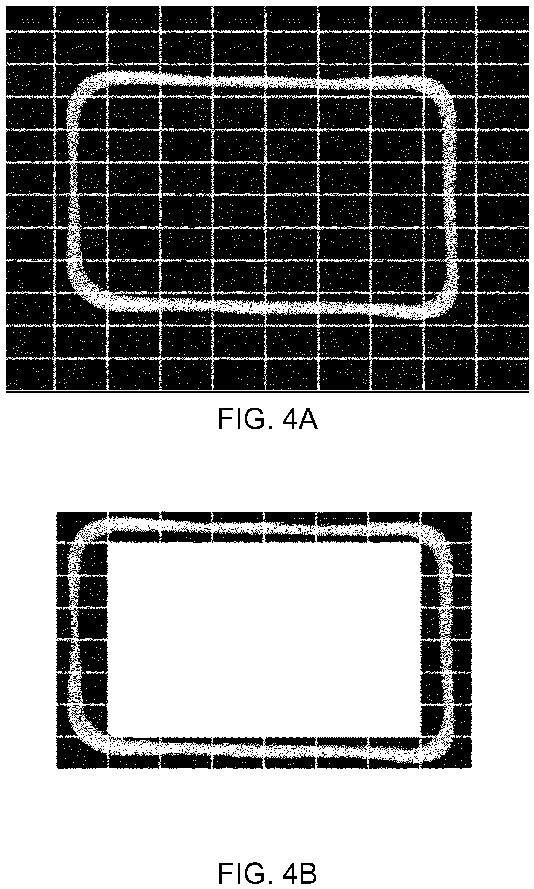

In a particular example, before step d) of extracting one or more descriptors, the image is subdivided into a plurality of segments as depicted in Figure 4. In this embodiment, the weld zone is identified and divided into segments or subareas, for example into 120 segments or subareas (not depicted in Figure 4).

For each subdivision or subarea, extraction of one or more descriptors, for example, the surface of the weld area and mean temperature of the weld area, or any combination of the previously described descriptors, is performed.

Sealing is optimal if the value of each of the descriptors of each of the segments is within the value limits or thresholds pre-established for the validation interval.

An exclusion zone can be defined for segments or subdivisons that are outside the weld zone, since they would not provide any information about the weld zone and would take computing power away from the control and processing system.

## Claims

1. A method for detecting defects in a weld of a container obtained by heat-sealing between two polymeric materials (1, 2), comprising the following steps:

   a) obtaining a thermographic image (I) of the weld, in an instant $t_1$, wherein $t_0 < t_1 < t_{max}$, where $t_0$ is the time instant in which the weld has been completed, $t_{max}$ is the time instant in which the weld acquires room temperature ($T_{amb}$) after cooling down from a welding temperature ($T_{sold}$), wherein $T_{sold} > T_{amb}$, and $t_1$ is the instant in which the thermographic image (I) is taken,
   b) rescaling the thermographic image (I) to increase the contrast between the zone corresponding to the weld and the rest of the container by correcting the value of each pixel based on the minimum and maximum temperature value of the image, obtaining as a result a reference image (R),
   c) identifying in the reference image (R) a weld area (S), binarizing the reference image and applying the binarized reference image by way of a mask on the thermographic image (I) for identifying said weld area (S) in the thermographic image (I),
   d) extracting at least one descriptor from the part of the thermographic image corresponding to the weld area (S), a descriptor being a measurable physical parameter, representative of the quality of the weld, and
   e) determining if the at least one extracted descriptor is within a validation interval defined by at least one threshold value,

   wherein the method additionally comprises one of the following options:

   i) correcting the thermographic image (I), before step d), at least the weld area (S), according to a value of the cooling of the polymeric materials (1, 2) taking into account the time elapsed between weld completion ($t_0$) and thermographic image capture ($t_1$), or
   ii) correcting, between steps d) and e), the value of the at least one extracted descriptor, according to the value of the cooling of the polymeric materials (1, 2) taking into account the time elapsed between weld completion ($t_0$) and thermographic image capture ($t_1$), obtaining a corrected descriptor value, or
   iii) correcting, before step e), the validation interval by correcting the at least one threshold value according to the cooling of the polymeric materials (1, 2) taking into account the time elapsed between weld completion ($t_0$) and thermographic image capture ($t_1$), wherein said value of the cooling of the polymeric materials (1, 2) is taken from a characteristic function (f) representing the cooling of the weld formed by these polymeric materials (1, 2) with respect to time.

2. The method for detecting defects according to claim 1, **characterized in that** in step b), rescaling the thermographic image (I) to increase the contrast comprises applying the following expression:

$$T_{adjusted} = \frac{T - T_{min}}{T_{max} - T_{min}} \times (2^R - 1)$$

where R is the resolution defined as the number of bits used in digitizing the thermographic image (I), $T_{max}$ is the maximum temperature of the thermographic image, $T_{min}$ is the minimum temperature of the thermographic image, T is the temperature in the thermographic image, and $T_{adjusted}$ is the temperature in the thermographic image rescaled to increase the contrast.

3. The method for detecting defects according to any of claims 1 or 2, where the characteristic function (f) is obtained from an experimental characteristic curve formed for the weld of the polymeric materials (1, 2) by means of recording temperature values of the weld during the time interval $[t_0, t_{max}]$ for a pre-established $T_{amb}$ and $T_{sold}$.

4. The method for detecting defects according to any of claims 1 or 2, where the characteristic function (f) is obtained from a mathematical model based on the law of cooling of materials, where the characteristic function (f) represents the cooling of polymeric materials (1, 2) with respect to time for any $T_{amb}$ and $T_{sold}$.

5. The method for detecting defects according to any one of the preceding claims, wherein the at least one extracted descriptor is a descriptor selected from the following:

   - surface of the weld area
   - width of the weld area
   - maximum width of the weld area
   - minimum width of the weld area
   - mean temperature of the weld area
   - minimum temperature of the weld area
   - maximum temperature of the weld area
   - perimeter of the weld area
   - interruption in the weld area
   - relations between these descriptors.

6. The method for detecting defects according to any one of the preceding claims, wherein when at least one of the extracted descriptors is outside the validation interval, the weld is identified as having a defect.

7. The method for detecting defects according to any one of the preceding claims, wherein before step d), the weld area of the thermographic image is subdivided into a plurality of segments, and steps d) and e) are applied on each of the segments, and wherein a defect is detected in the weld if a defect is detected in at least one of the segments.

8. A system (10) for detecting defects in a weld between two polymeric materials (1, 2) comprising:

   - a thermographic camera (11),
   - processing means (12), and
   - at least several time controlling means (13) **characterized in that** the processing means (12) are adapted for implementing a method for detecting defects according to any of claims 1 to 7.

9. The system (10) for detecting defects according to claim 8, further comprising:

   - a welding element (14) adapted for making the weld in the container,
   - a conveyance system (15) adapted for conveying the container from welding position $P_1$ to position P2 in which the thermographic image is taken,

   and wherein the time controlling means (13) are two position sensors (16, 17) adapted for determining the time elapsed between weld completion and the positioning of the container in the position where the thermographic image is taken.

**Patentansprüche**

1. Verfahren zum Detektieren von Fehlern in einer Schweißung eines Behälters, die durch Heißsiegeln zwischen zwei Polymermaterialien (1, 2) erhalten wird, das die folgenden Schritte aufweist:

a) Erhalten eines Thermografiebilds (I) der Schweißung zu einem Zeitpunkt $t_1$, wobei $t_0 < t_1 < t_{max}$, wobei $t_0$ der Zeitpunkt ist, zu dem die Schweißung abgeschlossen wurde, $t_{max}$ der Zeitpunkt ist, zu dem die Schweißung Raumtemperatur ($T_{amb}$) nach Abkühlung von einer Schweißtemperatur ($T_{sold}$) annimmt, wobei $T_{sold} > T_{amb}$, und $t_1$ der Zeitpunkt ist, zu dem das Thermografiebild (I) aufgenommen wird,

b) Reskalieren des Thermografiebilds (I), um den Kontrast zwischen der Zone in Entsprechung zur Schweißung und dem Rest des Behälters durch Korrigieren des Werts jedes Pixels auf der Grundlage des minimalen und maximalen Temperaturwerts des Bilds zu erhöhen, wobei als Ergebnis ein Referenzbild (R) erhalten wird,

c) im Referenzbild (R) erfolgendes Identifizieren eines Schweißbereichs (S), Binarisieren des Referenzbilds und Auflegen des binarisierten Referenzbilds mittels einer Maske auf dem Thermografiebild (I) zum Identifizieren des Schweißbereichs (S) im Thermografiebild (I),

d) Extrahieren mindestens eines Deskriptors aus dem Teil des Thermografiebilds in Entsprechung zum Schweißbereich (S), wobei ein Deskriptor ein messbarer physikalischer Parameter ist, der die Güte der Schweißung darstellt, und

e) Bestimmen, ob der mindestens eine extrahierte Deskriptor in einem Validierungsintervall liegt, das durch mindestens einen Schwellwert definiert ist,

wobei das Verfahren zusätzlich eine der folgenden Optionen aufweist:

i) vor dem Schritt d) erfolgendes Korrigieren des Thermografiebilds (I), mindestens des Schweißbereichs (S), gemäß einem Wert der Abkühlung der Polymermaterialien (1, 2) unter Berücksichtigung der Zeit, die zwischen Schweißabschluss ($t_0$) und Thermografiebildaufnahme ($t_1$) vergeht, oder

ii) zwischen den Schritten d) und e) erfolgendes Korrigieren des Werts des mindestens einen extrahierten Deskriptors gemäß dem Wert der Abkühlung der Polymermaterialien (1, 2) unter Berücksichtigung der Zeit, die zwischen Schweißabschluss ($t_0$) und Thermografiebildaufnahme ($t_1$) vergeht, wobei ein korrigierter Deskriptorwert erhalten wird, oder

iii) vor dem Schritt e) erfolgendes Korrigieren des Validierungsintervalls durch Korrigieren des mindestens einen Schwellwerts gemäß der Abkühlung der Polymermaterialien (1, 2) unter Berücksichtigung der Zeit, die zwischen Schweißabschluss ($t_0$) und Thermografiebildaufnahme ($t_1$) vergeht,

wobei der Wert der Abkühlung der Polymermaterialien (1, 2) einer charakteristischen Funktion (f) entnommen wird, die die Abkühlung der durch diese Polymermaterialien (1,2) gebildeten Schweißung im Hinblick auf die Zeit darstellt.

2. Verfahren zum Detektieren von Fehlern nach Anspruch 1, **dadurch gekennzeichnet, dass** im Schritt b) das Reskalieren des Thermografiebilds (I) um den Kontrast zu erhöhen ein Anwenden des folgenden Ausdrucks aufweist:

$$T_{adjusted} = \frac{T - T_{min}}{T_{max} - T_{min}} \times \left(2^R - 1\right)$$

wobei R die Auflösung in der Definition als Anzahl von Bits ist, die beim Digitalisieren des Thermografiebilds (I) verwendet werden, $T_{max}$ die maximale Temperatur des Thermografiebilds ist, $T_{min}$ die minimale Temperatur des Thermografiebilds ist, T die Temperatur im Thermografiebild ist und $T_{adjusted}$ die Temperatur im Thermografiebild ist, das zur Kontrasterhöhung reskaliert ist.

3. Verfahren zum Detektieren von Fehlern nach Anspruch 1 oder 2, wobei die charakteristische Funktion (f) anhand einer experimentellen Kennlinie erhalten wird, die für die Schweißung der Polymermaterialien (1, 2) mittels Aufzeichnung von Temperaturwerten der Schweißung während des Zeitintervalls $[t_0, t_{max}]$ für einen vorab festgelegten Wert $T_{amb}$ und $T_{sold}$ gebildet wird.

4. Verfahren zum Detektieren von Fehlern nach Anspruch 1 oder 2, wobei die charakteristische Funktion (f) anhand eines mathematischen Modells auf der Grundlage des Abkühlungsgesetzes von Materialien erhalten wird, wobei die charakteristische Funktion (f) die Abkühlung von Polymermaterialien (1, 2) im Hinblick auf die Zeit für jeden Wert

$T_{amb}$ und $T_{sold}$ darstellt.

**5.** Verfahren zum Detektieren von Fehlern nach einem der vorstehenden Ansprüche, wobei der mindestens eine extrahierte Deskriptor ein Deskriptor ist, der aus den Folgenden ausgewählt ist:

- Oberfläche des Schweißbereichs,
- Breite des Schweißbereichs,
- maximale Breite des Schweißbereichs,
- minimale Breite des Schweißbereichs,
- mittlere Temperatur des Schweißbereichs,
- minimale Temperatur des Schweißbereichs,
- maximale Temperatur des Schweißbereichs,
- Umfang des Schweißbereichs,
- Unterbrechung im Schweißbereich,
- Beziehungen zwischen diesen Deskriptoren.

**6.** Verfahren zum Detektieren von Fehlern nach einem der vorstehenden Ansprüche, wobei in dem Fall, dass mindestens einer der extrahierten Deskriptoren außerhalb des Validierungsintervalls liegt, die Schweißung als fehlerhaft identifiziert wird.

**7.** Verfahren zum Detektieren von Fehlern nach einem der vorstehenden Ansprüche, wobei vor dem Schritt d) der Schweißbereich des Thermografiebilds in mehrere Segmente unterteilt wird und die Schritte d) und e) auf jedes der Segmente angewendet werden und wobei ein Fehler in der Schweißung detektiert wird, wenn ein Fehler in mindestens einem der Segmente detektiert wird.

**8.** System (10) zum Detektieren von Fehlern in einer Schweißung zwischen zwei Polymermaterialien (1, 2), das aufweist:

eine Thermografiekamera (11),
eine Verarbeitungseinrichtung (12) und
mindestens mehrere Zeitsteuereinrichtungen (13),
**dadurch gekennzeichnet, dass** die Verarbeitungseinrichtung (12) zum Realisieren eines Verfahrens zum Detektieren von Fehlern nach einem der Ansprüche 1 bis 7 ausgebildet ist.

**9.** System (10) zum Detektieren von Fehlern nach Anspruch 8, das ferner aufweist:

ein Schweißelement (14), das zum Herstellen der Schweißung im Behälter ausgebildet ist,
ein Fördersystem (15), das zum Fördern des Behälters von einer Schweißposition $P_1$ zu einer Position P2 ausgebildet ist, an der das Thermografiebild aufgenommen wird,
und wobei die Zeitsteuereinrichtungen (13) zwei Positionssensoren (16, 17) sind, die zum Bestimmen der Zeit geeignet sind, die zwischen Schweißabschluss und der Positionierung des Behälters an der Position vergeht, an der das Thermografiebild aufgenommen wird.

**Revendications**

**1.** Un procédé de détection de défauts dans une soudure d'un récipient obtenu par thermoscellage entre deux matériaux polymériques (1, 2), comprenant les étapes suivantes :

a) le fait d'obtenir une image thermographique (I) de la soudure, en un instant $t_1$, $t_0 < t_1 < t_{max}$, où $t_0$ est l'instant au moment duquel la soudure a été achevée, $t_{max}$ est l'instant au moment duquel la soudure acquiert la température ambiante ($T_{amb}$) après refroidissement depuis une température de soudage ($T_{sold}$), où $T_{sold} > T_{amb}$, et $t_1$ est l'instant au moment duquel l'image thermographique (I) est prise,
b) le fait de traiter l'image thermographique (I) pour augmenter le contraste entre la zone correspondant à la soudure et le reste du récipient en corrigeant la valeur de chaque pixel en fonction de la valeur de température minimale et maximale de l'image, ceci ayant pour résultat d'obtenir une image de référence (R),
c) le fait d'identifier dans l'image de référence (R) une zone de soudure (S), de binariser l'image de référence et d'appliquer l'image de référence binarisée au moyen d'un masque sur l'image thermographique (I) pour identifier ladite zone de soudure (S) dans la image thermographique (I),

d) le fait d'extraire au moins un descripteur de la partie de l'image thermographique correspondant à la zone de soudure (S), un descripteur étant un paramètre physique mesurable, représentatif de la qualité de la soudure, et

e) le fait de déterminer si ledit au moins un descripteur extrait est situé dans un intervalle de validation défini par au moins une valeur de seuil,

le procédé comprenant en outre l'une des options suivantes :

i) le fait de corriger l'image thermographique (I), avant l'étape d), au moins dans la zone de soudure (S), en fonction d'une valeur du refroidissement des matériaux polymères (1, 2) en tenant compte du temps écoulé entre l'achèvement de la soudure ($t_0$) et la capture de l'image thermographique ($t_1$),
ou

ii) le fait de corriger, entre les étapes d) et e), la valeur dudit au moins un descripteur extrait, en fonction de la valeur du refroidissement des matériaux polymères (1, 2) en tenant compte du temps écoulé entre l'achèvement de la soudure ($t_0$) et la capture de l'image thermographique ($t_1$), permettant d'obtenir une valeur de descripteur corrigée, ou

iii) le fait de corriger, avant l'étape e), l'intervalle de validation en corrigeant ladite au moins une valeur de seuil en fonction du refroidissement des matériaux polymères (1, 2) en tenant compte du temps écoulé entre l'achèvement de la soudure ($t_0$) et la capture de l'image thermographique ($t_1$),

ladite valeur du refroidissement des matériaux polymères (1, 2) étant prise à partir d'une fonction caractéristique (f) représentant le refroidissement de la soudure formée par ces matériaux polymères (1, 2) en fonction du temps.

2.  Le procédé de détection de défauts selon la revendication 1, **caractérisé en ce qu'**à l'étape b), le traitement de l'image thermographique (I) pour augmenter le contraste consiste à appliquer l'expression suivante :

$$ T_{adjusted} = \frac{T - T_{min}}{T_{max} - T_{min}} \times (2^R - 1) $$

dans laquelle R est la résolution définie comme étant le nombre de bits utilisés pour numériser l'image thermographique (I), $T_{max}$ est la température maximale de l'image thermographique, $T_{min}$ est la température minimale de l'image thermographique, T est la température dans l'image thermographique, et $T_{adjusted}$ est la température dans l'image thermographique traitée pour augmenter le contraste.

3.  Le procédé de détection de défauts selon l'une quelconque des revendications 1 ou 2, dans lequel la fonction caractéristique (f) est obtenue à partir d'une courbe caractéristique expérimentale formée pour la soudure des matériaux polymères (1, 2) au moyen de valeurs de température d'enregistrement de la soudure pendant l'intervalle de temps [$t_0$, $t_{max}$] pour un $T_{amb}$ et un $T_{sold}$ préétablis.

4.  Le procédé de détection de défauts selon l'une quelconque des revendications 1 ou 2, dans lequel la fonction caractéristique (f) est obtenue à partir d'un modèle mathématique basé sur la loi de refroidissement de matériaux, la fonction caractéristique (f) représentant le refroidissement de matériaux polymères (1, 2) par rapport au temps pour tout $T_{amb}$ et $T_{sold}$.

5.  Le procédé de détection de défauts selon l'une quelconque des revendications précédentes, dans lequel ledit au moins un descripteur extrait est un descripteur choisi parmi les suivants :

- surface de la zone de soudure
- largeur de la zone de soudure
- largeur maximale de la zone de soudure
- largeur minimale de la zone de soudure
- température moyenne de la zone de soudure
- température minimale de la zone de soudure
- température maximale de la zone de soudure
- périmètre de la zone de soudure

- interruption dans la zone de soudure
- relations entre ces descripteurs.

**6.** Le procédé de détection de défauts selon l'une quelconque des revendications précédentes, dans lequel lorsqu'au moins un des descripteurs extraits est en dehors de l'intervalle de validation, la soudure est identifiée comme présentant un défaut.

**7.** Le procédé de détection de défauts selon l'une quelconque des revendications précédentes, dans lequel avant l'étape d), la zone de soudure de l'image thermographique est subdivisée en une pluralité de segments, et les étapes d) et e) sont appliquées sur chacun des segments, et dans lequel un défaut est détecté dans la soudure si un défaut est détecté dans au moins un des segments.

**8.** Un système (10) de détection de défauts dans une soudure entre deux matériaux polymériques (1, 2) comprenant :

- une caméra thermographique (11),
- des moyens de traitement (12), et
- au moins plusieurs moyens (13) de commande de temps

**caractérisé en ce que** les moyens de traitement (12) sont adaptés pour mettre en oeuvre un procédé de détection de défauts selon l'une quelconque des revendications 1 à 7.

**9.** Le système (10) de détection de défauts selon la revendication 8, comprenant en outre :

- un élément de soudage (14) adapté pour réaliser la soudure dans le récipient,
- un système de transport (15) adapté pour transporter le conteneur de la position de soudage $P_1$ à la position $P_2$ dans laquelle l'image thermographique est prise,

et dans lequel les moyens de commande de temps (13) sont deux capteurs de position (16, 17) adaptés pour déterminer le temps écoulé entre l'achèvement de la soudure et le positionnement du récipient dans la position dans laquelle l'image thermographique est prise.

FIG. 1

EP 3 128 323 B1

FIG. 2A

FIG. 2B

FIG. 2C

FIG. 2D

FIG. 2E

FIG. 3

FIG. 4A

FIG. 4B

FIG. 5

FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000088781 B
- WO 2006012194 A
- JP 2003307505 B